(19) Europäisches Patentamt
European Patent Office
Office européen des brevets

(11) Publication number: **0 031 705**
**B1**

(12) # EUROPEAN PATENT SPECIFICATION

(45) Date of publication of patent specification: **21.03.84**

(51) Int. Cl.[3]: **C 07 D 275/02,**
A 01 N 43/80 //C07C155/04,
C07D295/18

(21) Application number: **80304667.1**

(22) Date of filing: **22.12.80**

(54) **Substituted 3-isothiazolones, salts and metal salt complexes thereof, compositions containing them and their use for combating bacteria, algae and fungi.**

(30) Priority: **26.12.79 US 107006**

(43) Date of publication of application:
**08.07.81 Bulletin 81/27**

(45) Publication of the grant of the patent:
**21.03.84 Bulletin 84/12**

(84) Designated Contracting States:
**AT BE CH DE FR GB IT LI NL SE**

(56) References cited:
GB - A - 1 390 443
US - A - 4 105 431
US - A - 4 169 949

(73) Proprietor: **Rohm and Haas Company**
**Independence Mall West**
**Philadelphia, Pennsylvania 19105 (US)**

(72) Inventor: **Petigara, Ramesh Balubhai**
**2185 Stewart Drive**
**Hatfield Pennsylvania, 19440 (US)**

(74) Representative: **Wood, Peter Worsley Spencer et al,**
**Rohm and Haas Company Patent Department**
**Chesterfield House Bloomsbury Way**
**London WC1A 2TP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 031 705 B1

Substituted 3-isothiazolones, salts and metal salt complexes thereof, compositions containing them and their use for combating bacteria, algae and fungi

This invention concerns novel compounds which are substituted 3-isothiazolones, salts and metal salt complexes thereof, compositions containing the novel compounds and the use of the novel compounds for combating pests, notably bacteria, algae and fungi.

U.S. Patent Specification No. 4,105,431 discloses substituted 3-isothiazolones in which (a) the 2-position is substituted with $(C_1—C_8)$alkyl, $(C_3—C_6)$cycloalkyl, phenyl$(C_1—C_4)$alkyl or phenyl which is optionally substituted with one or more halogen atoms, (b) the 4-position is optionally substituted with halogen or $(C_1—C_4)$alkyl and (c) the 5-position is optionally substituted with halogen or $(C_1—C_4)$alkyl. This U.S. Patent Specification also discloses salts of the substituted 3-isothiazolones and U.K. Patent Specification No. 1,390,443 discloses metal salt complexes of the 3-isothiazolones. These prior art compounds are disclosed as having utility as algaecides, bactericides and fungicides.

The present invention provides compounds somewhat similar in structure to these prior art compounds but which are distinguished therefrom in that they have in the 5-position a substituent which is an (N,N-disubstituted thiocarbamoyl)thio or cycloamino group. More specifically the compounds of the invention are compounds of Formula I below, salts or metal salt complexes thereof,

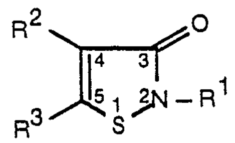

(I)

wherein

$R^1$ is $(C_1—C_8)$alkyl, $(C_3—C_6)$cycloalkyl, phenyl$(C_1—C_4)$alkyl or phenyl which is optionally substituted with up to 3 of the same or different halogen atoms (i.e. fluorine, chlorine, bromine and iodine);

$R^2$ is hydrogen, halogen or $(C_1—C_4)$alkyl; and

$R^3$ is a substituent; characterized in that $R^3$ is a group of the formula

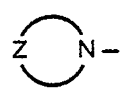

or a group of the formula

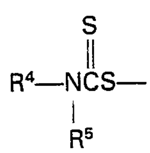

in which Z is $(—CH_2—)_n$, n being an integer of 4 to 6 and $R^4$ and $R^5$, which may be the same or different, is each selected from $(C_1—C_8)$alkyl and phenyl$(C_1—C_4)$alkyl or $R^4$ and $R^5$ are joined to form, together with the nitrogen atom to which they are attached a group of the formula

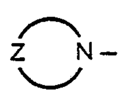

in which Z is as defined above.

The 3-isothiazolones, salts and metal salt complexes provided by the invention are of utility in that they have different biological activity spectra in comparison with the known isothiazolone derivatives mentioned above, and, in particular, many of the novel derivatives display activity in combating phytopathogenic fungi.

Typical $R^1$ groups are methyl, ethyl, n-propyl, isopropyl, n-butyl, n-hexyl, n-octyl, cyclopropyl, cyclobutyl, cyclopentyl, cyclohexyl, benzyl, phenethyl, phenyl-n-butyl, phenyl and phenyl substituted with 1 or 2 halogen atoms, particularly 1 or 2 chlorine or bromine atoms.

Typically $R^2$ represents hydrogen, fluorine, chlorine, bromine, methyl, ethyl, n-propyl, isopropyl, n-butyl and t-butyl.

Typical $R^3$ groups are pyrrolidinyl, piperidyl, (N,N-dimethylthiocarbamoyl)thio, (N,N-di-n-octylthiocarbamoyl)thio, (N-methyl-N-benzylthiocarbamoyl)thio, (N,N-dibenzylthiocarbamoyl)thio, [N,N-di(phenyl-n-butyl)thiocarbamoyl]thio, (N,N-tetramethylenethiocarbamoyl)thio and (N,N-hexamethylenethiocarbamoyl)thio.

A preferred sub-class of compounds, including salts and metal salts complexes, are those in which, in Formula I $R^1$ is $(C_1—C_8)$alkyl, benzyl, phenethyl or phenyl which is optionally substituted with

one or two of the same or different atoms selected from chlorine and bromine; $R^2$ is hydrogen, methyl or chlorine and $R^3$ is

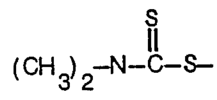 , 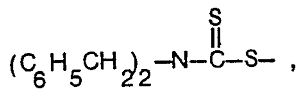 , 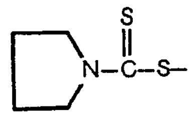 or 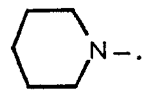.

The isothiazolones of Formula I can form salts with acids, in the manner described in U.S. Patent No. 4,105,431. Preparation of these biocidally active salts is readily achieved by reacting the appropriate 3-isothiazolone with an inorganic or organic acid which is preferably a strong acid. Typical acids which can be used include hydrochloric, nitric, sulfuric, maleic, succinic, hydrobromic, chlorosulfonic, chloroacetic, oxalic and p-toluenesulfonic. Separation of the salts from the reaction medium is accomplished by any conventional means.

Preferred metal salt complexes of the 3-isothiazolones of Formula I have the formula:

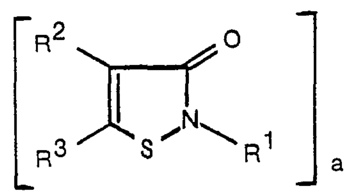 $M_bX_c$

wherein

$R^1$, $R^2$ and $R^3$ are as defined above,

M is a cation of a metal, such as barium, cadmium, calcium, chromium, cobalt, copper, iron, lead, magnesium, manganese, mercury, nickel strontium, tin or zinc;

X is an anion forming a compound with the cation M;

a is 1 or 2;

b is an integer which satisfies the valence of the anion X; and

c is an integer which satisfies the valence of the cation M.

Among the anions which X can represent are chloride, bromide, iodide, sulfate, nitrate, nitrite, acetate, chlorate, perchlorate, bisulfate, bicarbonate, oxalate, maleate, p-toluenesulfonate, carbonate and phosphate. M is typically a cation selected from Ba, Cd, Ca, Cr, Co, Cu, Fe, Pb, Mg, Mn, Hg, Ni, Sr, Sn and Zn. The preferred metals from which M is derived are calcium, copper, magnesium, manganese, nickel and zinc. Among the metal cations embraced by M are cationic complexes of the metal ions, including complexes with ammonia, simple organic amines, and various heterocyclic organic amines such as pyridines and pyrimidines.

A wide variety of metal nitrates and the metal nitrites can be used to stabilise solutions of the isothiazolones and generally any metal nitrate or nitrite which has appreciable solubility in the solution will exert a stabilizing effect. Among the useful metal nitrates are calcium nitrate, magnesium nitrate, copper nitrate, ferric nitrate, ferrous nitrate, nickel nitrate, zinc nitrate, barium nitrate, manganese nitrate, silver nitrate, cobalt nitrate, and the like. In a preferred embodiment of the invention, a metal nitrate is used to stabilize the isothiazolone solution. Surprisingly, other common metal salts, including carbonates, sulfates, chlorates, perchlorates, and chlorides are ineffective in stabilizing isothiazolones solutions.

Generally, the metal nitrate or nitrite is used to stabilize the isothiazolone solution in an amount of about 1 to about 30%, preferably about 15 to about 25%, by weight based on the weight of the solution. For example, in a 25% by weight solution of an isothiazolone, about 10 to about 30% by weight of the metal nitrate or nitrite will generally be sufficient to stabilize the solution against chemical decomposition. Of course, the amount of metal nitrate or nitrite needed to stabilize the solution will be partly dependent on the solvent, the isothiazolone and its concentration, the nitrate or nitrite used, the length of time the solution is to be kept, and other related factors.

The metal nitrates and metal nitrites are used to stabilize solutions of 3-isothiazolones in water and in polar organic solvents, including alcohols such as methanol, ethanol, propanol, butanol, ethylene glycol, propylene glycol, dipropylene glycol, polypropylene glycol, polyethylene glycol, 2-methoxyethanol, and the like. Among the applications in which solutions of isothiazolones are used are as water-cooling system microbicides, as preservatives for aqueous dispersions of organic polymers, as wood pulp white water slimicides, as cosmetic preservatives, as cutting oil, jet fuel, and heating oil preservatives, and the like. Solutions of isothiazolones are also often used applying an isothiazolone to a solid substrate, such as fabric, leather, or wood, as a preservative, especially in automated treating processes.

When solutions of metal salt complexes of the isothiazolones are to be used, it may be advantageous to prepare the complexes in situ by neutralization of a salt, such as the hydrochloride salt, of the appropriate isothiazolone with a basic metal compound, such as an oxide or carbonate. Optionally, up to about one half mole equivalent of a metal nitrate is added to these solutions, prior to the addition of the metal nitrate stabilizer.

The metal salt complexes can be made by the procedure of U.S. Patent Specification No. 4,067,878 for instance.

The compounds of Formula I, salts and metal salt complexes thereof, are fungicides which possess activity against assorted phytopathogenic fungi, particularly in agricultural loci. These compounds, salts and complexes can be used in concentrations of from about 50 to about 2000 ppm in a suitable carrier in controlling phytopathogenic fungi.

Generally, control of a living organism is achieved in accordance with this invention by contacting the organism with a compound of the invention in an amount which is effective to combat the organism. Any of the techniques known in the art can be employed to disseminate the compound in a manner so as to achieve the desired contact with the organism to be controlled. Spraying and fumigating are typical of such techniques.

The compounds of this invention can be utilized as algaecides, bactericides or fungicides in any locus and particularly in aqueous media, such as, for example, water-cooling systems, swimming pools, protective or decorative films, crop seeds, soil growing plants, paper pulp processes, aqueous polymer dispersions, water-based paints and the like. In addition, these compounds and/or compositions containing them can function as fabric, paper, wood, and leather preservatives, soap additives, cosmetic preservatives, sanitizing agents, such as in laundry wash water, detergents and soaps, preservatives for metal working compounds, such as cutting oil formulations, preservatives for fuels, fiber spin finish biocides, and the like. An example of cutting oil formulation is an emulsifiable liquid containing the biocide, water and an emulsifying agent.

In general, a locus subject to attack by microorganisms can be protected by incorporating into the locus a compound of the invention in an amount which is effective to combat the microorganisms. The term "contamination" is meant to include any attack by microorganisms which leads to a chemical or physical breakdown or disintegration of the locus as well as the proliferation of the microorganisms within the locus without an accompanying deleterious effect. The exact amount required will, of course, vary with the medium being protected, the microorganisms being controlled, the particular compositions being employed, and the like. Typically, in a liquid medium, excellent control is obtained when the compounds are incorporated in the range of 0.1 to 10,000 parts per million (ppm) or 0.00001 to 1% based on the weight of the medium. A range of 1 to 2000 ppm is prefered. The term "combat", as employed in the specification and claims of this application, is to be construed as the effect of any means which adversely affects the existence or growth of bacteria or fungi. This effect may comprise a complete killing action, eradication, arresting in growth, inhibition, reduction in number, or any combination of these effects.

The isothiazolones derivatives of the invention are especially useful as agricultural fungicides. As such, they are particularly valuable when formulated in a fungicidal composition. Such compositions normally comprise an agronomically acceptable carrier or diluent for the active ingredient. Where necessary or desirable, surfactants or other additives may be incorporated to give uniformly formulated mixtures. By "agronomically acceptable carrier" is meant any substance which can be utilized to dissolve, disperse or difuse the chemical incorporated therein without impairing the effectiveness of the active ingredient and which does no permanent damage to such environment as soil, equipment, and agronomic crops.

For use as fungicides, the isothiazolone derivatives may be formulated as wettable powders, emulsion concentrates, dusts, granular formulations, aerosols or flowable emulsifiable concentrates. The isothiazolone derivatives can also be dissolved in a water-miscible liquid such as ethanol, isopropanol or acetone, such solutions being easily extended with water. Details of formulation procedures for making agricultural fungicide formulation abound in the literature.

For use as bactericides and fungicides, dilute sprays can be supplied with active ingredient concentrations from about 23 to 9092 g per 378.5 litres which corresponds to about 0.05 to 20 pounds of the active ingredient per 100 U.S. gallons of carrier liquid spray. They are usually applied at 45.36 g to 4536 g per 378.5 litres and preferably at 56.7 g to 2268 g per 378.5 litres of carrier liquid spray. In more concentrated sprays, the active ingredient will be increased by a factor of 2 to 12. With dilute sprays, applications are usually made to the plants until run-off is achieved, whereas with more concentrated or low-volume sprays the materials are applied as mists.

The compounds of this invention can be utilized as the sole biocidal agents or they can be employed in conjunction with other fungicides, bactericides, algaecides, slimicides, mildewcides, insecticides, nematicides and other comparable pesticides.

The 3-isothiazolones of Formula I can be prepared by reacting a 5-chloro-3-isothiazolone of the formula

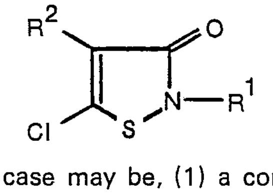 ($R^1$ and $R^2$ being as previously defined)

with, as the case may be, (1) a compound of the formula

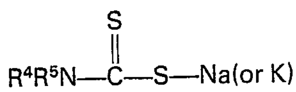

where $R^4$ and $R^5$ are as previously defined or (2) a compound of the formula

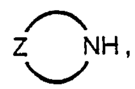

Z being as previously defined. These reactions are generally carried out in an inert solvent and typically at a temperature from about 10° to about 40°C.

An exemplary reaction scheme is given below.

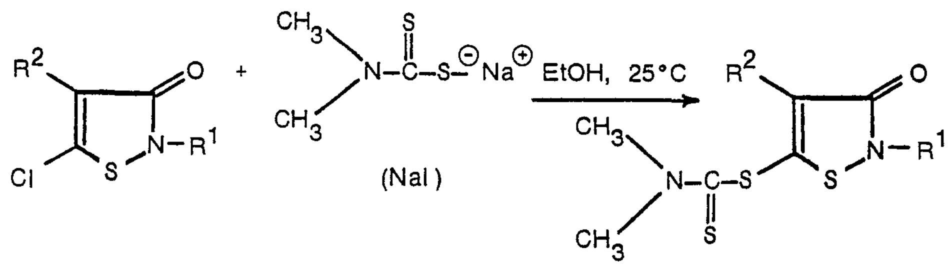


In place of sodium iodide in the above reaction, potassium iodide may be used. Other useful solvents include methanol, isopropanol, acetone, methylethyl ketone or a polar solvent, including water. The potassium salt of the dithiocarbamic acid is also useful. The temperature of the synthesis is from about 10° to about 40°C, preferably about room temperature.

An alkyl group in the 4-position of the isothiazolone ring facilitates the chlorine displacement reaction, whereas a chlorine in that position retarded the reaction. The order of ease of the displacement reaction is $CH_3$>H>Cl. In the absence of a 4-alkyl group some competing nucleophilic attack on S takes place, leading to isothiazolone ring cleavage. A phenyl group or a cycloalkyl group in the 2-position tends to favour this ring cleavage side reaction, whereas aralkyl and alkyl favours the chlorine displacement reaction.

Other reactions, giving rise to moderate yields of the desired product, are shown below.

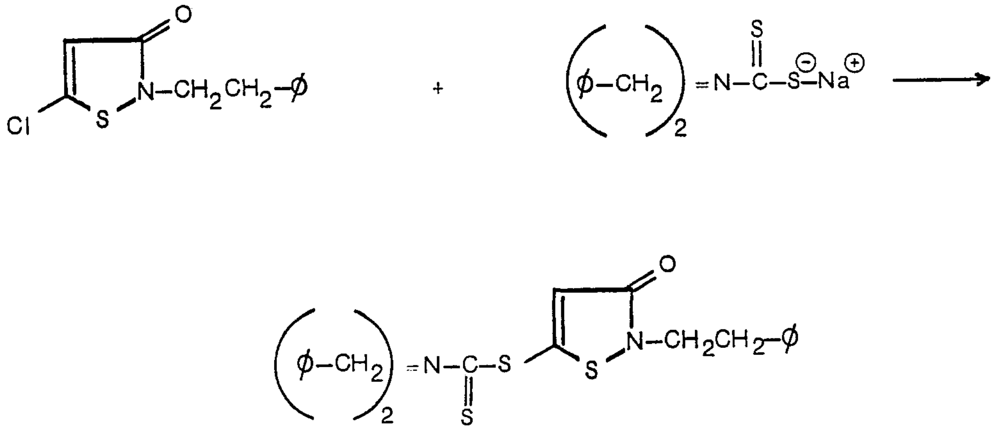

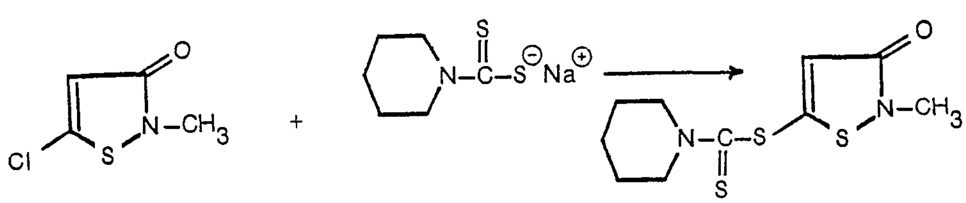

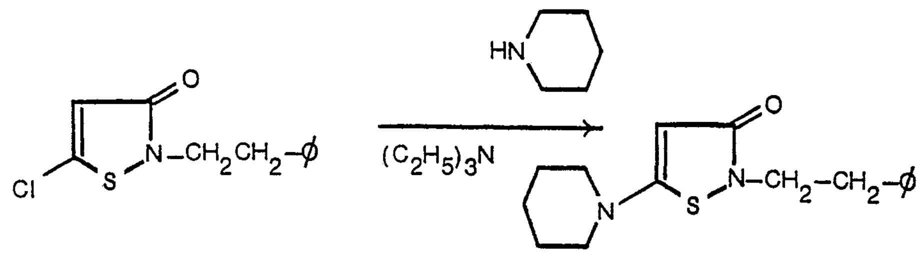


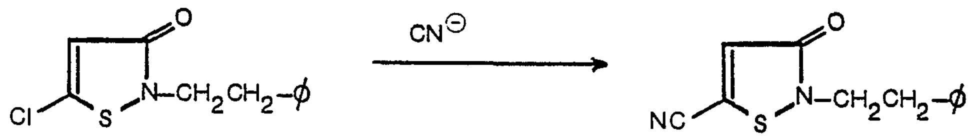


In the above reactions the starting 5-chloro isothiazolones can be prepared as disclosed in U.S. Patent No. 3,761,488 and 3,849,340 and as described in the publications (S.N. Lewis et al., *J. Heterocyclic Chemistry, 8,* 571(1971) and G.A. Miller et al., *ibid, 8,* 581(1971)).

The following are representative examples of procedures for obtaining the 5-substituted isothiazolone derivatives. In all instances the assigned structures were confirmed by elemental, IR and NMR analyses. Temperatures are expressed in degrees Celsius. Further details regarding these examples are to be found in Tables I and II which follow Example 15.

Example 1

2-Benzyl-5-(N,N-dimethylthiocarbamoyl)thio-4-isothiazolin-3-one

To a solution of 6.76 g (0.03 mole) of 2-benzyl-5-chloro-4-isothiazolin-3-one in 25 ml of ethanol was added a solution of 5.37 g (0.03 mole) of N,N-dimethyldithiocarbamic acid sodium salt dihydrate in 30 ml of ethanol followed by 0.1 g of sodium iodide. A yellow solid separated spontaneously. The mixture was stirred at room temperature for 3 hours, then cooled and filtered. The solid was triturated with 50 ml of water and recrystallized from 250 ml of boiling EtOH to give 5.6 g (60% yield) of the named product, mp 184—186°.

Example 2

5-(N,N-Dimethylthiocarbamoyl)thio-4-methyl-2-phenyl-4-isothiazolin-3-one

To a solution of 4.51 g (0.02 mole) of 5-chloro-4-methyl-2-phenyl-4-isothiazolin-3-one in 30 ml of warm ethanol was added a solution of 3.58 g (0.02 mole) of N,N-dimethyldithiocarbamic acid, sodium salt dihydrate in 20 ml of ethanol followed by 0.1 g of sodium iodide. The mixture was stirred several hours, cooled and filtered. The solid was triturated with water and dried to give 5.3 g (88% yield) of the named product, mp 158—160°.

Example 3

4-Chloro-5-(N,N-dimethylthiocarbamoyl)thio-2-phenethyl-4-isothiazolin-3-one

To a solution of 5.48 g (0.02 mole) of 4.5-dichloro-2-phenethyl-4-isothiazolin-3-one in 80 ml of ethanol was added 3.58 g (0.02 mole) of N,N-dimethyldithiocarbamic acid, sodium salt in 70 ml of ethanol followed by 0.15 g sodium iodide. The mixture was stirred for 2 hours, cooled, and filtered. The solid was crystallized from 150 ml of boiling EtOH to give 1.7 g (24% yield) of the named product, mp 125—127°.

Examples 4—11

These compounds, identified in Table I which follows were prepared in a manner similar to the procedures described in foregoing Examples 1—3.

Example 12

5-(N,N-Dibenzylthiocarbamoyl)thio-2-phenethyl-4-isothiazolin-3-one

*(a) N,N-Dibenzyldithiocarbamic acid sodium salt*

To a solution of 8.0 g (0.2 mole) of sodium hydroxide in 32 ml of water was added 39.4 g (0.2 mole) of dibenzyl amine. The mixture was cooled to 0° and to it was added 25.3 g (0.334 mole) of carbon disulfide. A solid separated spontaneously. The mixture was stirred at room temperature for 1 hour, and then filtered. The solid was triturated first with 100 ml of ether and then with 100 ml of benzene and dried to give 43.5 g (80% yield) of N,N-dibenzyldithiocarbamic acid sodium salt, mp 260°.

Anal Calcd. for $C_{15}H_{14}NS_2$ Na 2 1/2 $H_2O$

| | C | H | N | S |
|---|---|---|---|---|
| | 52.94 | 5.58 | 4.12 | 18.80 |
| Found: | 52.91 | 5.33 | 4.58 | 17.98 |

(b) to a solution of 4.8 g (0.02 mole) of 5-chloro-2-phenethyl-4-isothiazolin-3-one in 35 ml of ethanol was added 5.9 g (0.02 mole) of N,N-dibenzyldithiocarbamic acid sodium salt followed by 0.15 g sodium iodide. The mixture was concentrated *in vacuo* and the residue was treated with 300 ml of boiling hexane under reflux. The hexane solution decanted and cooled to give 0.63 g of the named product, mp 98—100°.

Anal Calcd. for $C_{26}H_{24}N_2OS_3$:

| | C | H | N | S |
|---|---|---|---|---|
| | 65.51 | 5.07 | 5.88 | 20.18 |
| Found: | 65.30 | 5.19 | 5.41 | 19.73 |

## Example 13

2-Methyl-5-[N,N-tetramethylenethiocarbamoyl]thio 4-isothiazolin-3-one

*(a) 1-Pyrrolidinecarbodithioic acid, sodium salt, dihydrate*

To a solution of 12.0 g (0.3 mole) NaOH in 30 ml of water was added 21.34 g (0.3 mole) of pyrroline. The mixture was cooled at 0° and to it 37.9 g (0.5 mole) of $CS_2$ was added dropwise. A white solid separated spontaneously. The mixture was stirred for 15 minutes and then treated with 150 ml of ether. The solid was collected by filtration, triturated with 150 ml of ether, and dried at 50°C in vacuum for 3 hours to give 55.2 g of the product mp 260°.

Anal. Calcd. for $C_5H_8NS_2Na\ 2H_2O$

| | C | H | N |
|---|---|---|---|
| | 29.27 | 5.85 | 6.83 |
| Found: | 29.20 | 5.75 | 7.52 |

(b) To a solution of 3.0 g (0.02 mole) of 5-chloro-2-methyl-4-isothiazolin-3-one in 30 ml of ethanol was added dropwise a solution of 4.1 g (0.02 mole) of 1-pyrrolidine carbodithioic acid, sodium salt dihydrate in 60 ml of ethanol. A solid separated spontaneously, which was collected by filtration and recrystallized from 200 ml of ethanol to give 1.9 g of a product. This was found to be a mixture of two products by TLC (silica gel, diisopropyl ether); Rf 0.0 (desired product) and Rf 0.43 (unknown). The mixture was separated on a dry column chromatograph (1.27 x 50.8 cm silica gel) using diisopropyl ether. The product near the origin was eluted with 100 ml of $CHCl_3$. The solvent was removed to give 1.3 g of the named product, mp 245—250°.

Anal. Calcd. for $C_9H_{12}N_2OS_3$:

| | C | H | N | S |
|---|---|---|---|---|
| | 41.45 | 4.61 | 10.77 | 36.92 |
| Found: | 41.89 | 4.61 | 10.82 | 36.14 |

## Example 14

5-Piperidino-2-phenethyl-4-isothiazolin-3-one

A mixture of 2.4 g (0.01 mole) of 5-chloro-2-phenethyl-4-isothiazolin-3-one, 0.85 g (0.01 mole) of piperidine, 1.0 g (0.01 mole) triethylamine and 0.1 g sodium iodide in 10 ml of $CHCl_3$ was heated under reflux for 18 hours. The mixture was washed with water, dried ($MgSO_4$) and concentrated to dryness to give 2.7 g of a liquid residue. This was purified by a dry column chromatography (4 x 51 cm, silica gel, diisopropyl ether) to give 1.0 g of a solid product, which was recrystallized from 100 ml of diisopropyl ether to give 0.6 g of the named product, mp 106—108°.

Anal. Calcd. for $C_{16}H_{20}N_2OS$:

| | C | H | N | S |
|---|---|---|---|---|
| | 66.63 | 6.99 | 9.71 | 11.12 |
| Found: | 66.54 | 7.20 | 9.67 | 11.02 |

TABLE I

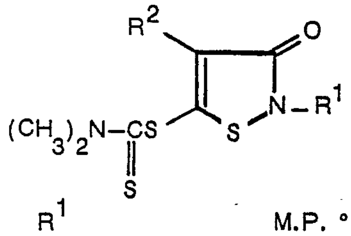

| Example | $R^2$ | $R^1$ | M.P. °C. | Recrystallization Solvent | % Yield |
|---|---|---|---|---|---|
| 1 | H | 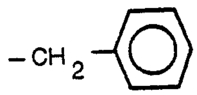 | 184–186 | EtOH | 60 |
| 2 | $CH_3$ | 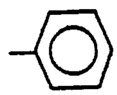 | 158–160 | none | 88 |
| 3 | Cl | 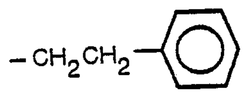 | 125–127 | EtOH | 24 |
| 4 | H | 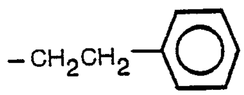 | 146.5–147.5 | none | 50 |
| 5 | H | $-CH_3$ | 191–193 | EtOH | 34 |
| 6 | H | $-C_8H_{17}$-*n* | 98–100 | $Me_2CO$ | 30 |
| 7 | $CH_3$ | 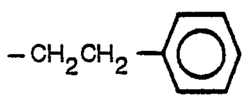 | 128–130 | EtOH | 60 |
| 8 | $CH_3$ | 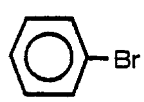 | 183–185 | none | 80 |
| 9 | $CH_3$ | 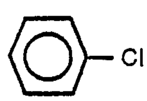 | 176–178 | none | 75 |
| 10 | $CH_3$ | 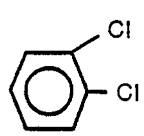 | 178–180 | $Me_2CO$ | 25 |
| 11 | $CH_3$ | 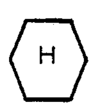 | 111–113 | $Me_2CO$ | 48 |

TABLE II

| Ex. | Empirical Formula | Elemental Analysis Calculated / Found C | H | N | S | Halogen |
|---|---|---|---|---|---|---|
| 1 | $C_{13}H_{14}N_2OS_3$ | 50.29<br>50.22 | 4.55<br>4.65 | 9.02<br>9.10 | 30.99<br>30.15 | – |
| 2 | $C_{13}H_{14}N_2OS_3$ | 50.29<br>49.89 | 4.54<br>4.50 | 9.02<br>9.13 | 30.98<br>30.26 | – |
| 3 | $C_{14}H_{15}ClN_2OS_3$ | 46.85<br>46.26 | 4.21<br>4.26 | 7.80<br>7.60 | 26.80<br>26.17 | 9.88<br>9.70 |
| 4 | $C_{14}H_{16}N_2OS_3$ | 51.85<br>51.64 | 4.94<br>5.05 | 8.64<br>8.47 | 29.63<br>29.22 | – |
| 5 | $C_7H_{10}N_2OS_3$ | 35.99<br>35.85 | 4.27<br>4.27 | 11.96<br>11.94 | 41.03<br>40.33 | – |
| 6 | $C_{14}H_{24}N_2OS_3$ | 50.57<br>50.36 | 7.27<br>7.46 | 8.42<br>8.76 | 28.93<br>28.31 | – |
| 7 | $C_{15}H_{18}N_2OS_3$ | 53.22<br>53.33 | 5.36<br>5.40 | 8.28<br>8.28 | 28.42<br>29.90 | – |
| 8 | $C_{13}H_{13}BrN_2OS_3$ | 40.10<br>39.98 | 3.27<br>3.32 | 7.19<br>7.31 | 24.71<br>23.52 | 20.52<br>21.30 |
| 9 | $C_{13}H_{13}ClN_2OS_3$ | 45.27<br>45.22 | 3.80<br>3.77 | 8.12<br>8.14 | 27.89<br>27.54 | 10.28<br>10.57 |
| 10 | $C_{13}H_{12}Cl_2N_2OS_3$ | 41.16<br>40.92 | 3.19<br>3.14 | 7.38<br>7.37 | 25.36<br>25.38 | 18.69<br>18.51 |
| 11 | $(C_{13}H_{20}N_2OS_3)_2.H_2O$ | 47.97<br>48.52 | 6.50<br>6.55 | 8.61<br>8.67 | 29.55<br>29.47 | –<br>– |

Most of the compounds of the foregoing examples were tested for activity against certain bacterial organisms and fungi including phytopathogenic fungi.

Antibacterial and antifungal activities were evaluated by the Serial Dilution Test (Broth Titer Test) wherein a series of broths containing varying dilutions of a test compound and an organism were halved starting with 1:1,000. The value recorded represents the maximum dilution in parts per million at which the compound under evaluation renders complete control of the organism. Pseudomonas aeruginosa, Staphylococcus aureus, and Escherichia coli were the bacterial organisms employed in this test, and the fungi employed were Aspergillus niger and Rhizopus stolonifera.

Activity of the compounds was determined against the following phytopathogenic fungi, barley net blotch (*Helminthosporium teres*) on barley plants (var. Wong), grey mold (*Botrytis fabae*) on faba beans (var. Vica faba), bean powdery mildew (*Erysiphe polygoni*) on bean plants (var. Dwarf Hort), grape downy mildew (*Plasmopora viticola*) on grape seedlings (var. Siebel 1000), rice blast (*Piricularia oryzae*) on rice plants (var. Nova 66), tomato late blight (*Phytophthora infestans*) on tomato seedlings (var. Rutgers), and wheat stem rust (Puccinia graminis f. sp. *tritici* race 15B-2) on wheat seedlings (var. Monon).

In the tests against phytopathogenic fungi evaluations were carried out by applying the compounds at a concentration of 300 ppm based on liquid carrier and spraying the plants (previously inoculated with spores of the fungi and incubated under standard conditions) to run off using a carrier dose of about 1400 litres/ha. The test conditions use are described in U.S. Patent Specification No. 4167576.

In general procedure was to take potted plants in proper condition of growth for susceptibility to the fungal disease to be evaluated, to spray these plants on a moving belt with the compound to be

evaluated, and allow them to dry. The plants were then inoculated with the fungal spores and then allowed to incubate until the disease has developed and the percent control is estimated. The test conditions employed are described in greater detail in U.S. Patent Specification No. 4,167,576.

Results of the above-described biological evaluations are set forth in Tables III and IV which follow. In these tables a dash indicates "not tested". Where, in Table IV, two ratings are given the righthand rating was obtained after subjecting the plant to 2.54 cm of simulated rain to give a measure of the persistency of the fungicide under evaluation.

TABLE III

Antibacterial and Antifungal Activities using Serial Dilution Test

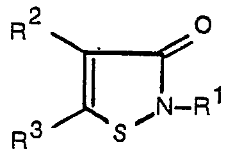

| Prod. of Ex. | $R^3$ | $R^2$ | $R^1$ | SDT, MIC in PPM[a] E | P | S | A | R |
|---|---|---|---|---|---|---|---|---|
| 1 | 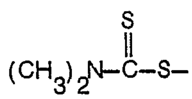 | H | 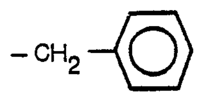 | | | > 500 | | |
| 2 | 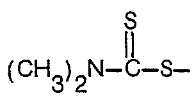 | $CH_3$ | 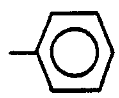 | 500 | >500 | – | 500 | – |
| 3 | 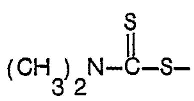 | Cl | 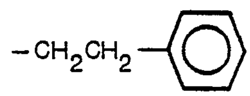 | | | > 500 | | |
| 4 | 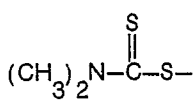 | H | 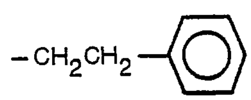 | | | > 500 | | |
| 5 | 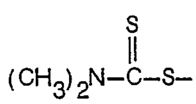 | H | $-CH_3$ | 500 | >500 | – | 125 | – |
| 6 | 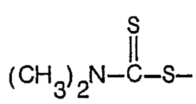 | H | $-C_8H_{17}$-*n* | – | – | – | – | – |
| 7 | 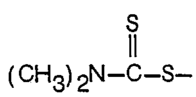 | $CH_3$ | 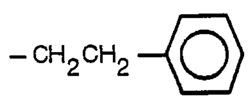 | | | > 500 | | |
| 8 | 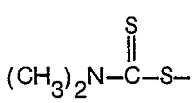 | $CH_3$ | 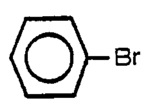 | 500 | 500 | 500 | 500 | 250 |
| 9 | 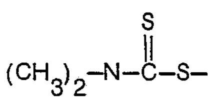 | $CH_3$ | 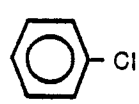 | 500 | 500 | 500 | 500 | 250 |

TABLE III (Continued)

Antibacterial and Antifugal Activities using Serial Dilution Test

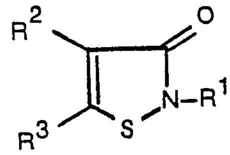

| Prod. of Ex. | $R^3$ | $R^2$ | $R^1$ | SDT, MIC in PPM[a] | | | | |
|---|---|---|---|---|---|---|---|---|
| | | | | E | P | S | A | R |
| 10 | 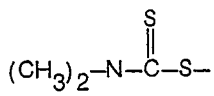 | $CH_3$ | 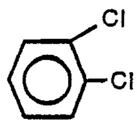 | 500 | 500 | 500 | 500 | 250 |
| 11 | 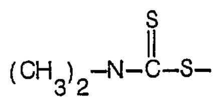 | $CH_3$ | 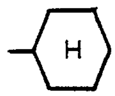 | 500 | 500 | 500 | 500 | 250 |
| 12 | 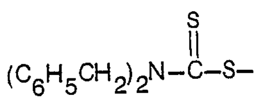 | H | 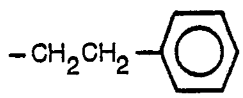 | – | – | – | – | – |
| 13 | 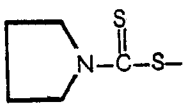 | H | $-CH_3$ | ——————> 500 —————— | | | | |
| 14 | 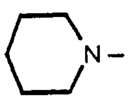 | H | 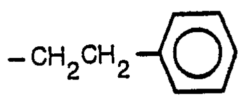 | 500 | 500 | 500 | 250 | 125 |

a *In vitro* serial dilution test (SDT), minimum inhibitory concentrations (MIC) against E = *E. coli*, P = *Ps. aeruginosa*, S = *Staphylococcus aureus*, A = *A. niger*, and R = *Rhizopus stolonifera* are given in parts per million of biocide.

TABLE IV

| Example | Concentration* (ppm) | Disease[b] Control Level[c] BH | BOT | BPM | GDM | RB | TLB | WSR |
|---|---|---|---|---|---|---|---|---|
| 1 | 300 | A | B | E | B | – | E | B |
| | 150 | E | B/B | – | C/C | A/E | – | C/C |
| | 75 | E | C | – | C | A | – | E |
| | 38 | E | C | – | D | A | – | E |
| 2 | 300 | E | B | E | E | – | E | C |
| | 150 | – | B/C | – | – | A/E | – | – |
| | 75 | – | B | – | – | B | – | – |
| | 38 | – | C | – | – | B | – | – |
| 3 | 300 | E | E | E | E | E | E | C |
| 4 | 300 | E | B | E | B | – | E | A |
| | 150 | – | A/C | – | A/A | A | – | E/E |
| | 75 | – | B | – | A | E | – | – |
| | 38 | – | B | – | B | E | – | – |
| 5 | 300 | A | B | E | B | – | E | B |
| | 150 | E | A/C | – | A/A | A/E | – | C/E |
| | 75 | E | A | – | A | A | – | E |
| | 38 | E | B | – | A | A | – | – |
| | 19 | E | – | – | C/E | A | – | – |
| 6 | 300 | E | E | E | E | – | – | A |
| 7 | 300 | E | E | E | E | – | E | A |
| | 150 | – | – | – | – | B/E | – | E |
| | 75 | – | – | – | – | B | – | E |
| | 38 | – | – | – | – | B | – | E |

* Concentration in carrier applied to run off at a dose of *ca* 1400 litres/ha.

TABLE IV (Continued)

| Example | Doses (ppm) | Disease[b] Control Level[c] | | | | | | |
|---|---|---|---|---|---|---|---|---|
| | | BH | BOT | BPM | GDM | RB | TLB | WSR |
| 8 | 300 | E | C | E | B | — | E | B |
| | 150 | — | — | — | A/B | A/E | — | C/C |
| | 75 | — | — | — | A | A | — | C |
| | 38 | — | — | — | B | A | — | C |
| 9 | 300 | E | B | E | A | A | E | B |
| | 150 | — | C/C | — | A/E | A/A | — | E |
| | 75 | — | C | — | E | A | — | E |
| | 38 | — | C | — | E | A | — | E |
| 10 | 300 | E | E | E | E | E | E | E |
| 11 | 300 | E | E | E | E | E | E | C |
| 12 | 300 | E | E | E | E | E | E | E |
| 13 | 300 | E | A | E | A | B | B | B |
| | 150 | — | A/E | — | A/E | A/E | A/E | C/E |
| | 75 | — | C | — | A | B | B | C |
| | 38 | — | C | — | A | B | E | C |
| 14 | 300 | E | E | E | E | A | E | A |
| | 150 | — | — | — | — | A/E | — | E |
| | 75 | — | — | — | — | A | — | E |
| | 38 | — | — | — | — | E | — | E |

[b] BH—Barley net blotch *(Helminthosporium teres)*, BOT—Chocolate spot of broad beans *(Botrytis cinerea)*, BPM = Bean powdery mildew *(Erisiphe polygoni)*, GDM = Grape downy mildew *(Plasmopora viticola)*, RB = Rice blast *(Piricularia oryzae)*, WSR = Wheat stem rust *(Puccinia graminis* f. sp. *tritici)*.

[c] Ratings: A = 97—100% Disease Control, B = 90—96%; C = 70—89%; D = 50—69%; E = <50%.

**Claims for the Contracting States: BE CH DE FR GB IT LI NL SE**

1. A compound of Formula I below, or a salt or metal salt complex thereof,

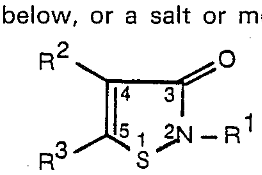

I

wherein

$R^1$ is $(C_1—C_8)$alkyl, $(C_3—C_6)$cycloalkyl, phenyl$(C_1—C_4)$alkyl or phenyl optionally substituted with up to 3 of the same or different halogen atoms;

$R^2$ is hydrogen, halogen or $(C_1—C_4)$alkyl; and

$R^3$ is a substituent; characterized in that $R^3$ is a group of the formula

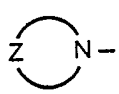

or a group of the formula

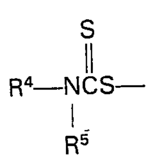

in which Z is $(-CH_2-)_n$, n being an integer of 4 to 6 and $R^4$ and $R^5$, which may be the same or different, is each selected from $(C_1-C_8)$alkyl and phenyl$(C_1-C_4)$alkyl or $R^4$ and $R^5$ are joined to form, together with the nitrogen atom to which they are attached, a group of the formula

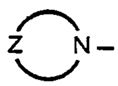

in which Z is as defined above.

2. A compound, salt or metal salt complex as claimed in Claim 1, wherein $R^1$ is $(C_1-C_8)$alkyl, benzyl, phenethyl or phenyl which is optionally substituted with one or two of the same or different atoms selected from chlorine and bromine; $R^2$ is hydrogen, methyl or chlorine and $R^3$ is

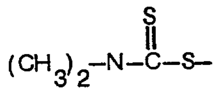 , 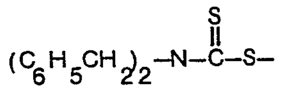 , 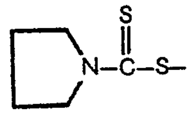 or 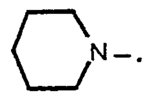.

3. A salt of a compound of Formula I as claimed in Claim 1 or 2, being a salt of an acid which is hydrochloric, nitric, sulfuric, hydrobromic, chlorosulfonic, chloroacetic, oxalic, maleic, succinic or p-toluenesulfonic.

4. A metal salt complex of the formula

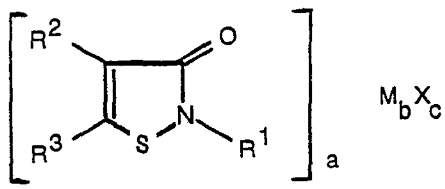

wherein $R^1$, $R^2$ and $R^3$ are as defined in Claim 1 or 2; a is 1 or 2; M is a cation selected from Ba, Cd, Ca, Cr, Co, Cu, Fe, Pb, Mg, Mn, Hg, Ni, Sr, Sn and Zn; X is an anion selected from chloride, bromide, iodide, sulfate, nitrate, nitrite, acetate, chlorate, perchlorate, bisulfate, bicarbonate, oxalate, maleate, p-toluenesulfonate, carbonate and phosphate; b is an integer which satisfies the valence of the anion X; and c is an integer which satisfies the valence of the cation M.

5. A composition for combating bacteria, algae or fungi which comprises a compound, salt or metal salt complex as claimed in any one of Claims 1 to 4 and a suitable carrier or diluent therefor.

6. A method of combating bacteria, algae, or fungi in a locus which comprises applying to the locus an effective amount of a compound, salt or metal salt complex as claimed in any one of Claims 1 to 4.

7. A method as claimed in Claim 6, wherein the locus is cutting oil formulation, water-cooling system, protective or decorative film, fibrous material, laundry wash water, crop seeds, a crop growth medium or growing plant.

**Claims for the Contracting State: AT**

1. A composition for combating bacteria, algae or fungi containing as active ingredient a 3-isothiazolone compound and a suitable carrier or diluent therefor, characterized in that said active ingredient is a compound of Formula I below, or a salt of metal salt complex thereof,

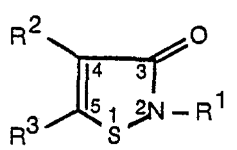

I

wherein

$R^1$ is $(C_1-C_8)$alkyl, $(C_3-C_6)$cycloalkyl, phenyl$(C_1-C_4)$alkyl or phenyl optionally substituted with up to 3 of the same or different halogen atoms;

$R^2$ is hydrogen, halogen or $(C_1-C_4)$alkyl; and

$R^3$ is a substituent; characterized in that $R^3$ is a group of the formula

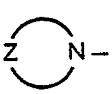

or a group of the formula

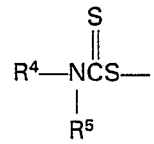

in which Z is $(-CH_2-)_n$, n being an integer of 4 to 6 and $R^4$ and $R^5$, which may be the same or different, is each selected from $(C_1-C_8)$alkyl and phenyl$(C_1-C_4)$alkyl or $R^4$ and $R^5$ are joined to form, together with the nitrogen atom to which they are attached, a group of the formula

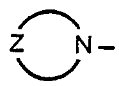

in which Z is as defined above.

2. A composition as claimed in Claim 1, wherein $R^1$ is $(C_1-C_8)$alkyl, benzyl, phenethyl or phenyl which is optionally substituted with one or two of the same or different atoms selected from chlorine and bromine; $R^2$ is hydrogen, methyl or chlorine and $R^3$ is

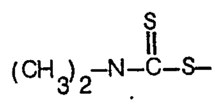 , 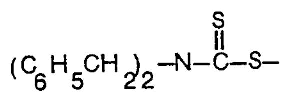 , 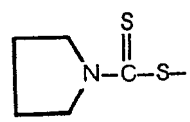 or 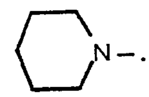

3. A composition as claimed in Claim 1 or 2, containing a salt of a compound of Formula I, being a salt of an acid which is hydrochloric, nitric, sulfuric, hydrobromic, chlorosulfonic, chloroacetic, oxalic, maleic, succinic or p-toluenesulfonic.

4. A composition as claimed in Claim 1, containing, as active ingredient, a metal salt complex of the formula

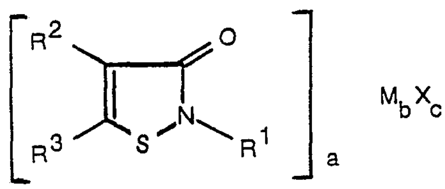

wherein $R^1$, $R^2$ and $R^3$ are as defined in Claim 1 or 2; a is 1 or 2; M is a cation selected from Ba, Cd, Ca, Cr, Co, Cu, Fe, Pb, Mg, Mn, Hg, Ni, Sr, Sn and Zn; X is an anion selected from chloride, bromide, iodide, sulfate, nitrate, nitrite, acetate, chlorate, perchlorate, bisulfate, bicarbonate, oxalate, maleate, p-toluenesulfonate, carbonate and phosphate; b is an integer which satisfies the valence of the anion X; and c is an integer which satisfies the valence of the cation M.

5. A method of combating bacteria, algae or fungi in a locus which comprises applying to the locus an effective amount of composition as claimed in any one of Claims 1 to 4.

6. A method as claimed in Claims 5, wherein the locus is a cutting oil formulation, water-cooling system, protective or decorative film, fibrous material, laundry wash water, crop seeds, a crop growth medium or growing plant.

**Revendications pour les Etats contractants: BE CH DE FR GB IT LI NL SE**

1. Un composé de formule (I) ci-dessous ou un de ses sels ou sels métalliques complexes:

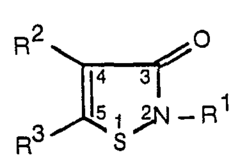

I

où

$R^1$ est un alcoyle $(C_1-C_8)$, un cycloalcoyle $(C_3-C_6)$, un phénylalcoyle $(C_1-C_4)$ ou un phényle éventuellement substitué par jusqu'à 3 atomes d'halogène semblables ou différents;

$R^2$ est un hydrogène, un halogène ou un alcoyle $(C_1-C_4)$; et

$R^3$ est un substituant; caractérisé en ce que $R^3$ est un groupe de formule

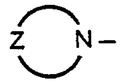

ou un groupe de formule:

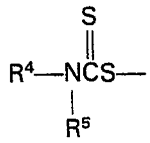

où Z est $(—CH_2—)_n$, n étant un entier de 4 à 6 et $R^4$ et $R^5$, qui peuvent être semblables ou différents, sont choisis chacun parmi un alcoyle $(C_1—C_8)$ et un phénylalcoyle $(C_1—C_4)$ ou $R^4$ et $R^5$ sont unis pour former avec l'atome d'azote auquel ils sont fixés un groupe de formule

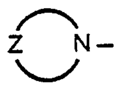

où Z est comme défini ci-dessus.

2. Un composé, sel ou sel métallique complexe comme revendiqué dans la revendication 1, où $R^1$ est un alcoyle $(C_1—C_8)$, un benzyle, un phénéthyle ou un phényle qui est éventuellement substitué par 1 ou 2 atomes semblables ou diférents choisis parmi le chlore et le brome: $R^2$ est un hydrogène, un méthyle ou un chlore, et $R^3$ est:

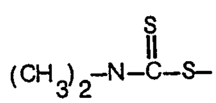 , 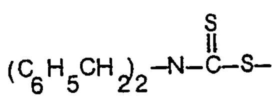 , 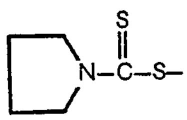 or 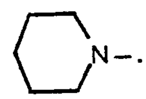

3. Un sel d'un composé de formule (I) comme revendiqué dans la revendication 1 ou 2, qui est un sel d'un acide qui est l'acide chlorhydrique, nitrique, sulfurique, bromhydrique, chlorosulfonique, chloroacétique, oxalique, maléique, succinique ou p-toluènesulfonique.

4. Un sel métallique complexe de formule:

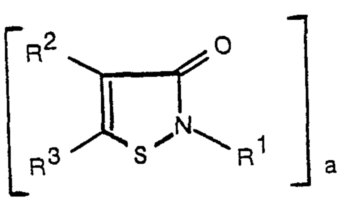 $M_bX_c$

où $R^1$, $R^2$ et $R^3$ sont comme défini dans la revendication 1 ou 2; a est 1 ou 2; M est un cation choisi parmi Ba, Cd, Ca, Cr, Co, Cu, Fe, Pb, Mg, Mn, Hg, Ni, Sr, Sn et Zn; X est un anion choisi parmi un chlorure, bromure, iodure, sulfate, nitrate, nitrite, acétate, chlorate, perchlorate, bisulfate, bicarbonate, oxalate, maléate, p-toluènesulfonate, carbonate et phosphate; b est un entier qui satisfait à la valence de l'anion X; et c est un entier qui satisfait à la valence du cation M.

5. Une composition pour lutter contre les bactéries, les algues ou les champignons, qui comprend un composé, sel ou sel métallique complexe comme revendiqué dans l'une quelconque des revendications 1 à 4 et un support ou diluant appropriés de celui-ci.

6. Un procédé pour lutter contre les bactéries, les algues ou les champignons en un site, qui comprend l'application au site d'une quantité efficace d'un composé, sel ou sel métallique complexe comme revendiqué dans l'une quelconque des revendications 1 à 4.

7, Un procédé comme revendiqué dans la revendication 6, dans lequel le site est une composition d'huile de coupe, un système de refroidissment par l'eau, une pellicule protectrice ou décorative, une matière fibreuse, une eau de lessive, des semences, un milieu de développement d'une culture ou une plante en croissance.

**Revendications pour l'Etat contractant: AT**

1. Une composition pour lutter contre les bactéries, les algues ou les champignons contenant comme ingrédient actif un composé de 3-isothiazolone et un support ou diluant appropriés de celui-ci, caractérisée en ce que ledit ingrédient actif est un composé de formule (I) ci-dessous ou un de ses sels ou sels métalliques complexes:

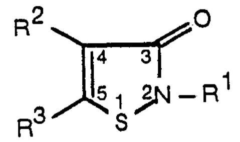

I

où

$R^1$ est un alcoyle ($C_1$—$C_8$), un cycloalcoyle ($C_3$—$C_6$), un phénylalcoyle ($C_1$—$C_4$) ou un phényle éventuellement substitué par jusqu'à 3 atomes d'halogène semblables ou différents;

$R^2$ est un hydrogène, un halogène ou un alcoyle ($C_1$—$C_4$); et

$R^3$ est un substituant; caractérisé en ce que $R^3$ est un groupe de formule

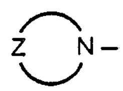

ou un groupe de formule:

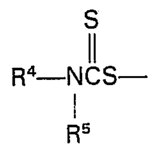

où Z est (—$CH_2$—)$_n$, n étant un entier de 4 à 6 et $R^4$ et $R^5$, qui peuvent être semblables ou différents, sont choisis chacun parmi un alcoyle ($C_1$—$C_8$) et un phénylalcoyle ($C_1$—$C_4$) ou $R^4$ et $R^5$ sont unis pour former avec l'atome d'azote auquel ils sont fixés un groupe de formule

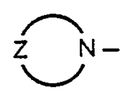

où Z est comme défini ci-dessus.

2. Une composition comme revendiqué dans la revendication 1, dans laquelle $R^1$ est un alcoyle ($C_1$—$C_8$), un benzyle, un phénéthyle ou un phényle qui est éventuellement substitué par 1 ou 2 atomes semblables ou différents choisis parmi le chlore et le brome; $R^2$ est un hydrogène, un méthyle ou un chlore et $R^3$ est:

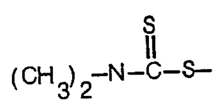 , 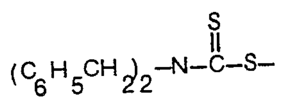 , 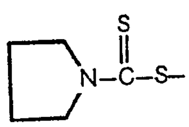 ou 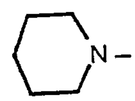 .

3. Une composition comme revendiqué dans la revendication 1 ou 2, contenant un sel d'un composé de formule (I), qui est un sel d'un acide qui est l'acide chlorhydrique, nitrique, sulfurique, bromhydrique, chlorosulfonique, chloroacétique, oxalique, maléique, succinique ou p-toluènesulfonique.

4. Une composition comme revendiqué dans la revendication 1, contenant comme ingrédient actif un sel métallique complexe de formule:

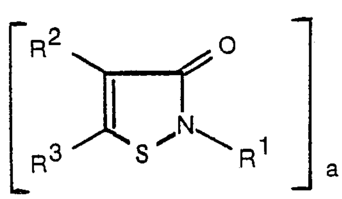 $M_bX_c$

où $R^1$, $R^2$ et $R^3$ sont comme défini dans la revendication 1 ou 2; a est 1 ou 2; M est un cation choisi parmi Ba, Cd, Ca, Cr, Co, Cu, Fe, Pb, Mg, Mn, Hg, Ni, Sr, Sn et Zn; X est un anion choisi parmi un chlorure, bromure, iodure, sulfate, nitrate, nitrite, acétate, chlorate, perchlorate, bisulfate, bicarbonate, oxalate, maléate, p-toluènesulfonate, carbonate et phosphate; b est un entier qui satisfait à la valence de l'anion X; et c est un entier qui satisfait à la valence du cation M.

5. Un procédé de lutte contre les bactéries, les algues ou les champignons en un site qui comprend l'application au site d'une quantité efficace d'une composition comme revendiqué dans l'une quelconque des revendications 1 à 4.

6. Un procédé comme revendiqué dans la revendication 5, dans lequel le site est une composition d'huile de coupe, un système de refroidissement par l'eau, une pelicule protectrice ou décorative, une matière fibreuse, une eau de lessive, des semences, un milieu de développement d'une culture ou une plante en croissance.

**Patentansprüche für die Vertragsstaaten: BE CH DE FR GB IT LI NL SE**

1. Verbindung der Formel I oder eines Salzes oder Metallsalzkomplexes davon

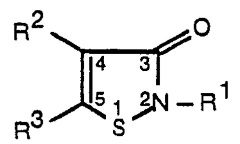

I

worin

$R^1$ für $(C_1—C_8)$Alkyl, $(C_3—C_6)$Cycloalkyl, Phenyl$(C_1—C_4)$Alkyl oder Phenyl, gegebenenfalls substituiert mit bis zu drei gleichen oder verschiedenen Halogenatomen, steht,

$R^2$ Wasserstoff, Halogen oder $(C_1—C_4)$Alkyl ist und

$R^3$ einen Substituent bedeutet, dadurch gekennzeichnet, daß $R^3$ eine Gruppe der Formel

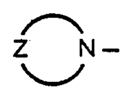

oder eine Gruppe der Formel

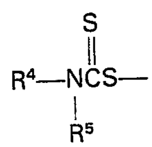

ist, wobei Z für $(—CH_2—)_n$ steht, n eine ganze Zahl von 4 bis 6 ist, und $R^4$ und $R^5$, die gleich oder verschieden sein können, aus $(C_1—C_8)$Alkyl und Phenyl $(C_1—C_4)$Alkyl ausgewählt sind, oder $R^4$ und $R^5$ miteinander verbunden sind und zusammen mit dem Stickstoffatom, mit dem sie verknüpft sind, eine Gruppe der Formel

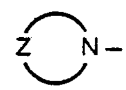

bilden, worin Z die vorstehend angegebene Bedeutung besitzt.

2. Verbindung, Salz oder Metallsalzkomplex nach Anspruch 1, dadurch gekennzeichnet, daß $R^1$ für $(C_1—C_8)$Alkyl, Benzyl, Phenethyl oder Phenyl, gegebenenfalls substituiert mit einem oder zwei gleichen oder verschiedenen Atomen, ausgewählt aus Chlor und Brom, steht, $R^2$ Wasserstoff, Methyl oder Chlor ist und $R^3$

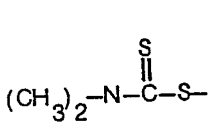 , 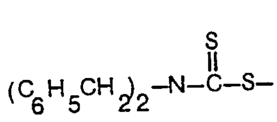 , 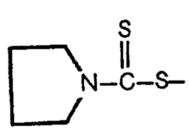 oder 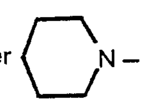

ist.

3. Salz einer Verbindung der Formel I gemäß Anspruch 1 oder 2, dadurch gekennzeichnet, daß es sich um ein Salz einer Säure handelt, die aus Chlorwasserstoff, Salpetersäure, Schwefelsäure, Bromwasserstoffsäure, Chlorsulfonsäure, Chloressigsäure, Oxalsäure, Maleinsäure, Bernsteinsäure oder p-Toluolsulfonsäure besteht.

4. Metallsalzkomplex der Formel

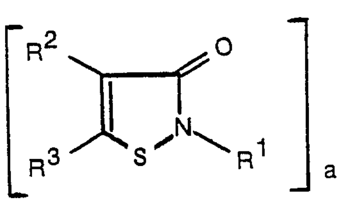

$M_bX_c$

worin $R^1$, $R^2$ und $R^3$ die in Anspruch 1 oder 2 angegebenen Bedeutungen besitzen, a 1 oder 2 ist, M ein Kation ist, ausgewählt aus Ba, Cd, Ca, Cr, Co, Cu, Fe, Pb, Mg, Mn, Hg, Ni, Sr, Sn sowie Zn, X ein Anion ist, ausgewählt aus Chlorid, Bromid, Jodid, Sulfat, Nitrat, Nitrit, Acetat, Chlorat, Perchlorat, Bisulfat, Bicarbonat, Oxylat, Maleat, p-Toluolsulfonat, Carbonat und Phosphat, b eine ganze Zahl ist, welche der Wertigkeit des Anions X entspricht, und c einer ganzen Zahl, die der Wertig keit des Kations M entspricht.

5. Mittel zur Bekämpfung von Bakterien, Algen oder Pilzen, gekennnzeichnet durch eine Verbindung, ein Salz oder einen Metallsalzkomplex nach einem der Ansprüche 1 bis 4 sowie einen geeigneten Träger oder ein geeignetes Verbindungsmittel dafür.

6. Verfahren zum Bekämpfen von Bakterien, Algen oder Pilzen an einer Stelle, dadurch gekennzeichnet, daß auf die Stelle eine wirksame Menge einer Verbindung, eines Salzes oder eines Metallsalzkomplexes gemäß einem der Ansprüche 1 bis 4 aufgebracht wird.

7. Verfahren nach Anspruch 6, dadurch gekennzeichnet, daß die Stelle eine Schneideölformulierung, ein Wasserkühlsystem, ein schützender oder dekorativer Film, ein faserartiges Material, Wäschereiwaschwasser, ein Nutzpflanzensaatgut, ein Nutzpflanzenwachstumsmedium oder eine wachsende Pflanze ist.

**Patentansprüche für den Vertragsstaat: AT**

1. Mittel zur Bekämpfung von Bakterien, Algen oder Pilzen, die als Wirkstoff eine 3-Isothiazolonverbindung und einen geeigneten Träger oder ein geeignetes Verdünnungsmittel dafür enthalten, dadurch gekennzeichnet, daß der Wirkstoff eine Verbindung der folgenden Formel I oder ein Salz oder ein Metallsalzkomplex davon ist

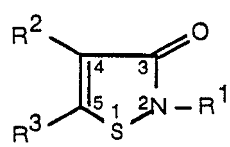

I

worin

$R^1$ für $(C_1—C_8)$Alkyl, $(C_3—C_6)$Cycloalkyl, Phenyl$(C_1—C_4)$Alkyl oder Phenyl, gegebenenfalls substituiert mit bis zu drei gleichen oder verschiedenen Halogenatomen, steht,

$R^2$ Wasserstoff, Halogen oder $(C_1—C_4)$Alkyl ist und

$R^3$ einen Substituenten bedeutet, wobei sich das Mittel weiter dadurch auszeichnet, daß $R^3$ eine Gruppe der Formel

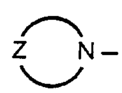

oder eine Gruppe der Formel

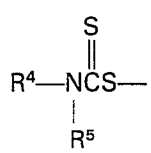

ist, wobei Z für $(—CH_2—)_n$ steht, n eine ganze Zahl von 4 bis 6 ist, und $R^4$ und $R^5$, die gleich oder verschieden sein können, aus $(C_1—C_8)$Alkyl und Phenyl $(C_1—C_4)$Alkyl ausgewählt sind, oder $R^4$ und $R^5$ miteinander verbunden sind und zusammen mit dem Stickstoffatom, mit dem sie verknüpft sind, eine Gruppe der Formel

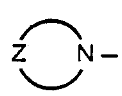

bilden, worin Z die vorstehend angegebene Bedeutung besitzt.

2. Mittel nach Anspruch 1, dadurch gekennzeichnet, daß $R^1$ für $(C_1—C_8)$Alkyl, Benzyl, Phenethyl oder Phenyl steht, gegebenenfalls substituiert mit einem oder zwei gleichen oder verschiedenen Atomen, ausgewählt aus Chlor und Brom, steht, $R^2$ Wasserstoff, Methyl oder Chlor ist und $R^3$

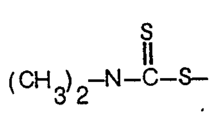 , 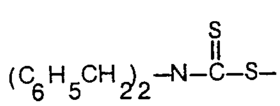 , 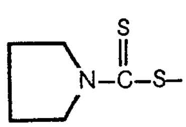 oder 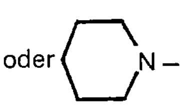.

bedeutet.

3. Mittel nach Anspruch 1 oder 2, dadurch gekennzeichnet, daß es ein Salz einer Verbindung der Formel I enthält, das aus einem Salz einer Säure besteht, die aus Chlorwasserstoffsäure, Salpetersäure, Schwefelsäure, Bromwasserstoffsäure, Chlorsulfonsäure, Chloressigsäure, Oxalsäure, Maleinsäure, Bernsteinsäure oder p-Toluolsulfonsäure besteht.

4. Mittel nach Anspruch 1, dadurch gekennzeichnet, daß es als Wirkstoff einen Metallsalzkomplex der Formel

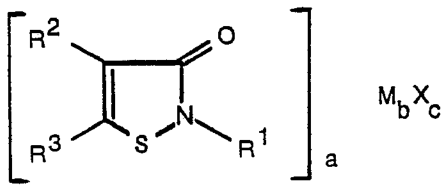

enthält, worin $R^1$, $R^2$ und $R^3$ die in Anspruch 1 oder 2 angegebenen Bedeutungen besitzen, a 1 oder 2 ist, M ein Kation ist, ausgewählt aus Ba, Cd, Ca, Cr, Co, Cu, Fe, Pb, Mg, Mn, Hg, Ni, Sr, Sn sowie Zn, X ein Anion ist, ausgewählt aus Chlorid, Bromid, Jodid, Sulfat, Nitrat, Nitrit, Acetat, Chlorat, Perchlorat, Bisulfat, Bicarbonat, Oxalat, Maleat, p-Toluolsulfonat, Carbonat und Phosphat, b eine ganze Zahl ist, die der Wertigkeit des Anions X entspricht, und c eine ganze Zahl ist, die der Wertigkeit des Kations M entspricht.

5. Verfahren zur Bekämpfung von Bakterien, Algen oder Fungi an einer Stelle, dadurch gekennzeichnet, daß auf die Stelle eine wirksame Menge eines Mittels gemäß einem der Ansprüche 1 bis 4 aufgebracht wird.

6. Verfahren nach Anspruch 5, dadurch gekennzeichnet, daß die Stelle eine Schneideölformulierung, ein Wasserkühlsystem, ein schützender oder dekorativer Film, ein faserartiges Material, ein Wäschereiwaschwasser, ein Nutzpflanzensaatgut, ein Nutzpflanzenwachstumsmedium oder eine wachsende Pflanze ist.